# EUROPEAN PATENT APPLICATION

(11) **EP 2 000 081 A1**
(43) Date of publication of application: **10.12.2008**
(21) Application number: 07465003.7
(22) Date of filing: 07.06.2007
(51) Int. Cl.: A61B 5/00, G01B 9/02, G01N 21/47

(54) **Apparatus for optical frequency domain tomography with adjusting system**

(71) Applicant: OPTOPOL Technology Spolka Akcyjna, 42-400 Zawiercie (PL)
(72) Inventor: Wojdas, Pawel, 42-400, Zawiercie (PL); Slusarczyk, Grzegorz, Chorzow 41-500, Chorzow (PL)
(74) Representative: Hudy, Ludwik

(57) **Abstract**

In an apparatus for imaging of objects by applying optical frequency domain tomography with an adjusting system that is provided with at least one device (170) for spectral analysis of a light beam with a dispersion device (171), a set of optical elements (172), a detection device (173) of the spectrum, the adjusting system for setting a relative position of photosensitive elements (174) of the detection device (173) and a spectrum image has at least one actuator acting on the dispersion device (171) and/or the set of optical elements (172) and/or the detection device (173) or a displaceable element of the dispersion device (171) and/or the set of optical elements (172) and/or the detection device (173) and movement of which causes the relative displacement between each other of at least one photosensitive element of the detection device of the spectrum and the spectrum image of the resultant light beam (270) until sufficient parameters of registration of the spectrum are achieved.

## Description

The present invention relates to an apparatus for optical frequency domain tomography with an adjusting system and a method for adjusting an apparatus for optical frequency domain tomography as well as an adjusting system of apparatus for optical frequency domain tomography.

Optical tomography is an imaging technique of objects and their internal structure that is based on analysis of a signal created from a light reference beam and a light object beam, which is back-scattered by object internal structures and interferences with the light reference beam and forms a resultant light beam. The resultant light beam is directed onto a dispersion device, for example a diffraction grating, and then is registered by a detection device or a spectrum recorder, for example, a matrix of photosensitive elements used in a linear CCD camera. The electrical signal in a form of a digital signal generated by the matrix is transmitted to a calculation unit and information about the axial structure of the object being examined is received by means of numerical calculation in the calculation unit, for instance by means of a PC.

Apparatuses to produce imaging of objects by means of optical tomography are known in numerous variants. However, independent from the variant, the accuracy of imaging of objects depends on adjusting the apparatus to work conditions and regulation carried out after out of tuning of the apparatus due to vibration during transport, change of temperature and new work condition of the apparatus. Nowadays used regulation systems are hand maintained, not precise, time consuming and need to be maintained by users that have been trained in the field of the imaging of objects by means of optical tomography.

The idea of the invention is in an apparatus for imaging of objects by applying optical frequency domain tomography and provided with an adjusting system for setting a relative position of photosensitive elements and a spectrum image, and comprising at least one device for spectral analysis of a light beam with a dispersion device, a set of optical elements, a detection device of the spectrum, in which the adjusting system is an automatically controlled device causing a relative displacement of at least one photosensitive element of the detection device of the spectrum and the spectrum image between each other, and comprising at least one actuator acting on the dispersion device and/or the set of optical elements and/or the detection device or a displaceable element of the dispersion device and/or the set of optical elements and/or the detection device and moving of which causes the relative displacement of at least one photosensitive element of the detection device of the spectrum and the spectrum image of the resultant light beam between each other.

Preferably, the relative displacement of at least one photosensitive element of the detection device of the spectrum and the spectrum image between each other takes place in a plane situated perpendicularly to propagation direction of the resultant light beam.

It is advantageous when the actuator acting on the dispersion device and/or the set of optical elements and/or the detection device or a displaceable element of the dispersion device and/or the set of optical elements and/or the detection device is a mechanism comprising at least one servomechanism controlled by electric signal, and a movable element of the servomechanism is connected mechanically to the dispersion device and/or the set of optical elements and/or the detection device or a displaceable element of the dispersion device and/or the set of optical elements and/or the detection device.

The actuator acting on the dispersion device and/or the set of optical elements and/or the detection device or a displaceable element of the dispersion device and/or the set of optical elements and/or the detection device can be a mechanism comprising at least one element connected to the dispersion device and/or the set of optical elements and/or the detection device or a displaceable element of the dispersion device and/or the set of optical elements and/or the detection device and the element is displaceabled by magnetic or electromagnetic field or a force evoked by pressure of fluid, gas or gas mixture, and is connected to the dispersion device and/or the set of optical elements and/or the detection device or to a displaceable element of the dispersion device and/or the set of optical elements and/or the detection device.

The idea of the invention is also a system for adjusting an apparatus for imaging of objects by applying optical frequency domain tomography provided with at least one device for spectral analysis of a light beam with a dispersion device, a set of optical elements and a detection device of the spectrum, which is an automatically controlled device causing a relative displacement of at least one photosensitive element of the detection device of the spectrum and the spectrum image between each other and comprising at least one actuator acting on the dispersion device and/or the set of optical elements and/or the detection device or a displaceable element of the dispersion device and/or the set of optical elements and/or the detection device and movement of which causes the relative displacement of at least one photosensitive element of the detection device of the spectrum and the spectrum image between each other.

Furthermore, the idea of the invention is a method for adjusting apparatus for optical frequency domain tomography provided with an adjusting system, at least one device for spectral analysis of a light beam formed from a reference light beam and an object light beam, a dispersion device for splitting a resultant light beam into a spectrum of the resultant light beam, a set of optical elements for forming and/or directing the resultant light beam and a detection device of the spectrum with at least one photosensitive element for registering the spectrum of resultant light beam, in which at least one photosensitive element of the detection device of the resultant light beam and an image of the spectrum is displaced relatively each other using at least one actuator acting on the dispersion device and/or the set of optical elements and/or the detection device or a displaceable element of the dispersion device and/or the set of optical elements and/or the detection device and movement of which causes the relative displacement of at least one photosensitive element of the detection device of the spectrum and the spectrum image of the resultant light beam between each other.

It is favorable that when displacing at least one photosensitive element of the detection device of the resultant light beam and the image of the spectrum relatively each other using the actuator, the detection device and a line of the image of the spectrum is brought to cross each other, and that a point of change-over is determined at the detection device and direction of rotation of at least one photosensitive element relatively to the image of the resultant light beam or rotation of the image of the resultant light beam relatively to at least one photosensitive element is changed after crossing the point of change-over by the line of the image of the spectrum, and that changing the direction of rotation is being done until parameters of registration of the spectrum determined by a manufacturer of the apparatus for optical frequency domain tomography or recognized by a user as sufficient for registration of the spectrum are achieved.

It is also favorable that the displacing of at least one photosensitive element relatively to the image of the resultant light beam is being done until an electric signal is generated by the detection device, and after determining a point of change-over on the detection device, direction of rotation of at least one photosensitive element of the detection device relatively to the image of the resultant light beam or rotation of the image of the resultant light beam relatively to at least one photosensitive element of the detection device is changed at every crossing the point of change-over by the line of the image of the spectrum, and changing of the direction of rotation is being done until a curve of light intensity / or energy registered by the detection device versus wave length A achieves a shape corresponding to a shape of a curve of light intensity / or energy of the light emitted by the light source versus wave length A or area under the curve of light intensity / or energy registered by the detection device versus wave length A achieves area corresponding to the area under the curve of light intensity / emitted by the light source after taking into account scattering loss.

The invention will now be described by way of example and with reference to the accompanying drawings in which:
Figs. 1 - 5 show block diagrams of different arrangements of apparatus for imaging of objects applying spectral optical coherence tomography with an adjusting system;
Figs. 6A, 6B and 6C show location of a spectrum and a matrix at different adjustment states of apparatus for imaging of objects;
Fig. 7 shows a flow diagram of an adjusting procedure of the apparatus for imaging of objects;
Fig. 8 shows a flow diagram of a preliminary roughly adjusting procedure;
Fig. 9 shows a flow diagram of a fine adjusting procedure;
Fig. 10 shows a flow diagram of an adjusting finish procedure by applying one servomechanism;
Fig. 11 shows a flow diagram of an adjusting step procedure;
Fig. 12 shows a flow diagram of an adjusting step procedure with a change of matrix movement direction; and
Fig. 13 shows a flow diagram of an adjusting step procedure with a change of servomechanism.

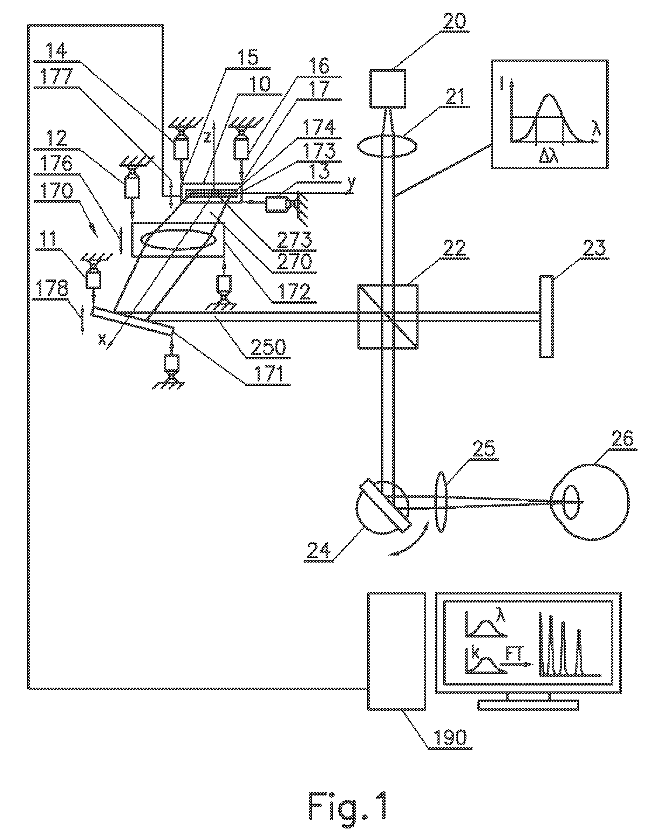

Fig. 1 shows an apparatus for optical frequency domain tomography according to present invention, called also an apparatus for optical frequency coherence tomography, provided with an adjusting system. The apparatus for optical frequency domain tomography comprises, among others, a light source 20 emitting a light formed by a collimating lens 21 and then split into two arms by a beam splitter 22. In the object arm the light, directed by a transverse scanning mirror 24 and an object lens 25, penetrates the interior of an object 26, is back-scattered by its internal structures and returns to the beam splitter 22. Simultaneously, light in the reference arm is reflected from a fixed or moveable reference mirror 23 and also returns to the beam splitter 22, in which a resultant beam is created and is registered by a spectrometer 170. In the spectrometer 170, the resultant beam 250 is directed onto a diffraction grating 171, which splits the resultant beam into a spectrum 270 modulated by interference fingers and registered by a detection device 173, for example by a matrix of photosensitive elements 174. An electrical signal, generated by the detection device, for example linear CCD camera type AViiVA M2CL2014 made by Atmel, is transmitted to a calculation unit 190. Information about the axial structure of the object being examined (single image line - A-scan) is received by means of numerical calculation in the calculation unit 190, for instance by means of a PC. After recording a first cross-section, the beam is traversed in the y axis (perpendicular to the x axis) forming a further trace in direction x. As a final result a three dimensional reconstruction of the object is created.

An adjusting system, with which the apparatus for optical frequency domain tomography is provided, and which can be a device 176 to align the optical elements 172, a device 177 to control a position of the detection device 173 as well as a device 178 to control a position of the spectrometer relative to the resultant light beam 250, comprises, among others, at least one working unit or actuator, for instance a servomechanism 11, 12, 13, 14, 16, for example servomechanism type HS-645MG manufactured by Hitec, USA, a moving element of which is mechanically coupled with a moving element of one of above mentioned devices 176, 177 and 178 causing relative moving of at least one photosensitive element 174 of the detection device 173, called also a spectrum recorder, and a spectrum 273 of the resultant light beam 250 in yz-plane situated perpendicularly to direction of the resultant light beam propagation, in this case, situated perpendicularly to x-axis. The moving element of one of above mentioned devices 176, 177 and 178 can be an end of a supporting element 10 of the detection device, a point of support or an articulated joint 15, 17, with which the working element or doing element of the servomechanism 14, 16, is coupled, and displacement or shift of which is automatically controlled by an electrical signal sent, for instance from the calculation unit 190. The moving element can be also an element displaced by magnetic or electromagnetic field or a force caused by pressure of liquid, gas or a gas mixture.

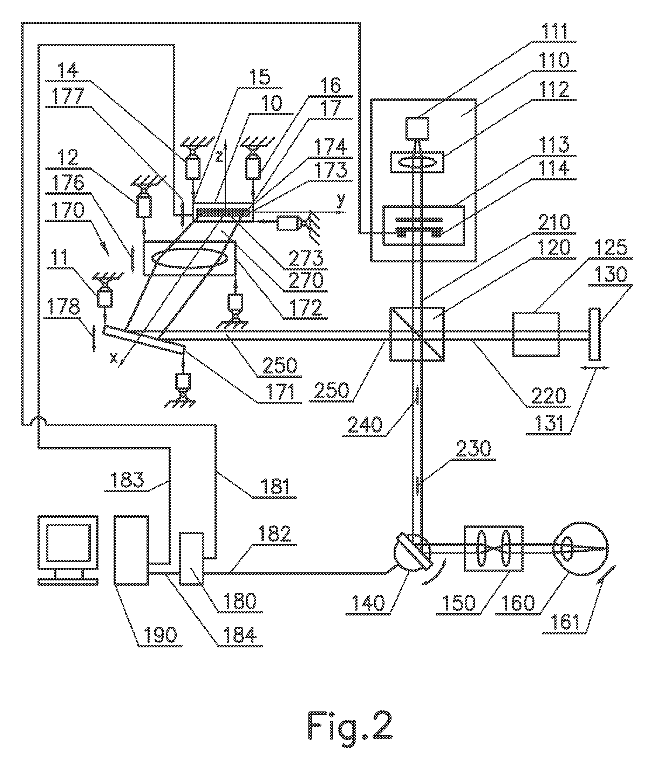

Fig. 2 shows other embodiment of the apparatus for optical frequency domain tomography, which comprises, among others, an optical frequency comb generator 110 emitting a light beam 210 consisting of discrete spectral components equidistantly distributed in optical frequency domain. The generating device or the optical frequency comb generator 110 is provided with a light source 111 emitting a light with low temporal coherence and high spatial coherence with a range or a spectral band *Δλ* and a central wave length *λ₀,* an optical element 112, such as a collimating lens, and an adjustable or tuned or scanning Fabry-Perot interferometer 113. One of the mirrors of the adjustable Fabry-Perot interferometer 113 is mounted on a positioning device 114 to control positions of the components of the optical frequency comb and/or to adjust distances between the components of the component set to distances between photosensitive elements 174 of the detection device 173, for example a matrix of photosensitive elements. The light beam 210, generated by the generator 110 of the optical components or the optical frequency comb, passes through a beam splitter 120, which splits the light beam 210 into at least one reference light beam 220 and at least one object light beam 230. The reference light beam 220 passes through a system of optical elements 125 to compensate for dispersion in both arms of the apparatus, and is directed to a reference mirror 130 and is then returned by the reference mirror 130. As it returns, the reference light beam again passes through the system of optical elements 125 for compensation of dispersion and again reaches the beam splitter 120.

The object light beam 230 is directed onto an object by an optical system 140 for changing beam direction, in which at least one mirror is mounted on a pivoted mechanism, and by a set of optical elements 150 that forms the object light beam 230, dependant on the selected/specific application and/or polarizes the object light beam 230. For instance, for examination of the eye fundus a set of two lenses in a telescopic arrangement may be applied. A returning object light beam 240, with a circular, elliptical or linear cross-section, backscattered or reflected from the internal structures of the object 160 with an adjusting system 161, is collected by the set of optical elements 150 and is directed to the beam splitter 120 by the optical system 140 for changing beam direction. The object light beam 240 returning from the object and the reference light beam 220 reflected from the reference mirror 130 with an adjusting device 131 is assembled in the beam splitter 120 into a combined light beam 250, which is then analysed by the spectrometer 170 consisting of a diffraction grating 171, a system of optical elements 172 forming an arrangement for adjusting the distances between the components of the component set to the distances between the photosensitive elements 174 of the matrix, which may be a component of a multi-element detection device 173, for example a CCD device. In the spectrometer 170, the resultant light beam 250 is transformed into a resultant spectrum 260 by a device separating spatially wavelength components such as the diffraction grating 171, and then passes through the system of optical elements 172 and is then displayed on the matrix of photosensitive elements 173, which transforms the displayed spectrum 270 into electrical signals for further analysis. Like to the embodiment shown in Fig. 1, the adjusting system, with which the apparatus for optical frequency domain tomography is provided, can be the device 176 to align the optical elements 172, the device 177 to control a position of the detection device 173 as well as the device 178 to control a position of the spectrometer relative to the resultant light beam 250, comprises, among others, at least one working unit, for instance the servomechanism 11, 12, 13, 14, 16, the moving element of which is mechanically coupled with the moving element of one of above mentioned devices 176, 177 and 178 causing relative moving of at least one photosensitive element 174 of the detection device 173 and the spectrum 273 of the resultant light beam.

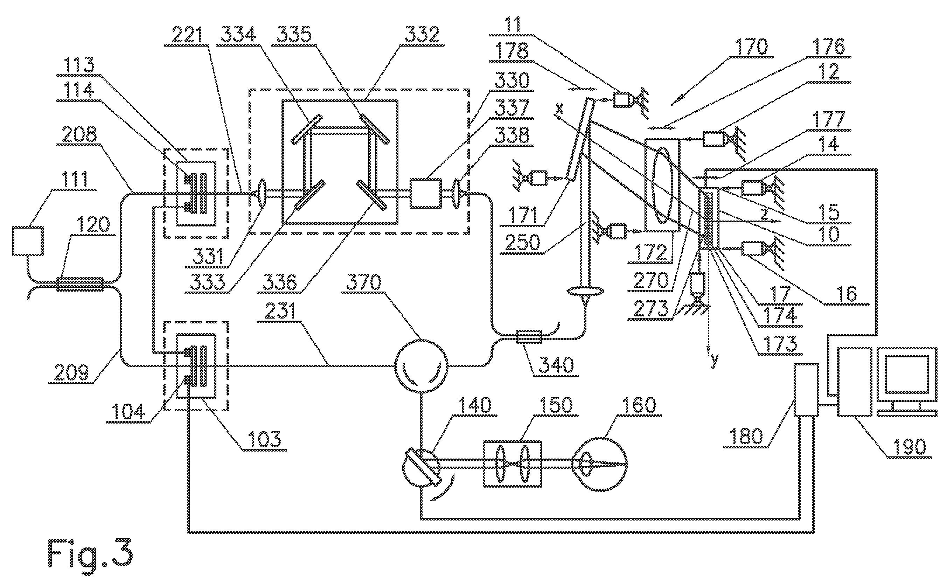

Fig. 3 shows a fiber-optic configuration of the apparatus for optical frequency domain tomography, with the light source 111, the Fabry-Perot interferometers 103, 113 that are fitted with a positioning device 104, 114 and that are aligned with the reference light beam 208 and the object light beam 209. In other embodiment one of Fabry-Perot interferometers can be placed in front of a fiber-coupler 120 in the way of light beam. In the embodiment of the apparatus for optical frequency coherence tomography, which is constructed using fibre technology, the reference light beam passes through an optical arrangement 330 that forms the reference light beam 221 or passes the group of optical elements which compensate or correct dispersion in both arms of the interferometer and/or passes a light-polarizer, and next, the reference light beam is directed to a fiber-coupler 340. The optical arrangement 330 may contain an optical element 331 such as a collimator, a delay system 332, possibly a combination of four mirrors 333 - 336, a group of optical components 337 for compensation of dispersion in both arms of the apparatus and an optical element 338, such as a focussing lens. In turn, the object light beam 230 is directed onto the object by a linear optical 3-way circulator 370 and the optical system 140 for changing beam direction, and by a set of optical elements 150 that forms the object light beam 230, dependant on the selected application, like in the configuration in the open optics. The optical system 140 for changing beam direction may also be provided with an adjustable light-filter that can be placed in other place, to restrict the intensity of the object light beam during the detection of the background spectrum. The resultant light beam 250, formed from the reference and object light beams, is directed onto the diffraction grating 171, from which as the spectrum 270 is imaged onto the detection device 173.

Additionally, the apparatus for optical frequency domain tomography within the spectral frequency band contains a control system 180 to generate signals for the generating device of the optical components or the optical frequency comb generator to control the positions of the optical components in the light beam as well as a control signal to align the optical system 140, for instance a set of galvanometric mirrors, for changing light beam direction. The control signal sets the galvanometric mirrors that direct the object light beam onto the object 160 to be examined in a given position.

The apparatus for optical frequency domain tomography within the spectral frequency band comprises, beside the calculation unit 190, the adjusting system of the apparatus for optical frequency domain tomography, which can be the device 176 to align the optical elements 172, the device 177 to control a position of the detection device 173 as well as the device 178 to control a position of the spectrometer relative to the resultant light beam 250, comprises, among others, at least one working unit, for instance the servomechanism 11, 12, 13, 14, 16, the moving element of which is mechanically coupled with the moving element of one of above mentioned devices 176, 177 and 178 causing relative moving of at least one photosensitive element 174 of the detecting device 173 and the spectrum 273 of the resultant light beam.

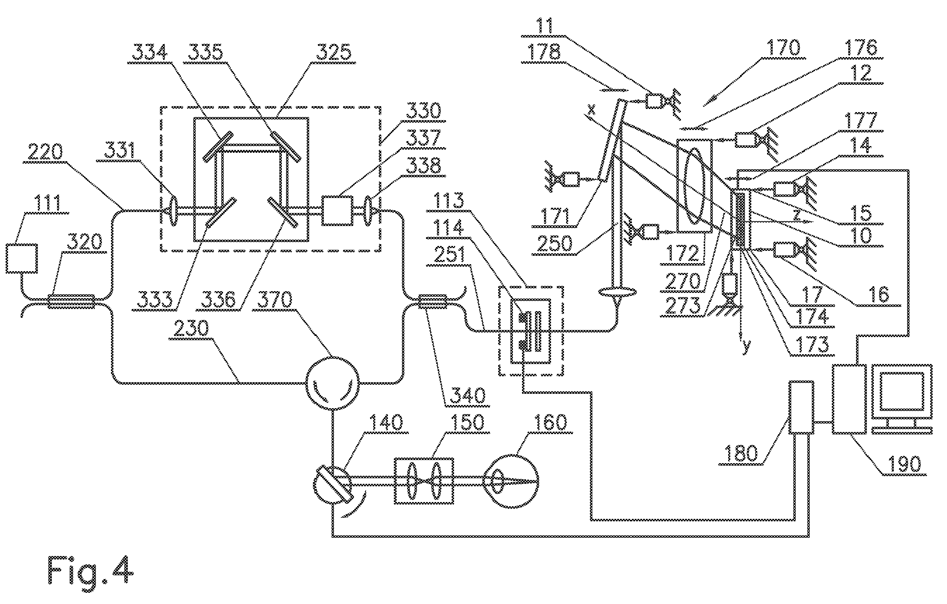

The presented invention shown in Fig. 4 as an embodiment of an apparatus for optical frequency coherence tomography comprises a light source 111, a fiber-coupler 320, an optical arrangement 330 with an optical element 331 such as a collimator, a delay system 335, possibly a combination of four mirrors 333 - 336, a group of optical components 337 for compensation of dispersion in both arms of the apparatus and an optical element 338, such as a focusing lens for focusing the reference light beam 220. In turn, the object light beam 230 is directed onto the object by a linear optical 3-way circulator 370 and the optical system 140 for changing beam direction, and by a set of optical elements 150 that forms the object light beam 230, dependant on the selected application, like in the configuration in the open optics. The reference and object light beams are directed to the fiber-coupler 320, and then as a beam 251 is directed to the adjustable Fabry-Perot interferometer controlled by the positioning device 114 and to the spectrometer 170, from which as a resultant light beam is directed onto the diffraction grating 171, from which as the spectrum 270 is imaged onto the detection device 173.

Additionally, the apparatus for optical frequency domain tomography within the spectral frequency band contains the control system 180, a calculation unit 190 as well as an adjustment device for adjusting the apparatus for optical frequency domain tomography, which can be the device 176 to align the optical elements 172, the device 177 to control a position of the detection device 173 and/or the device 178 to control a position of the spectrometer relative to the resultant light beam 250, and which comprises, among others, at least one working unit or an actuator, for instance the servomechanism 11, 12, 13, 14, 16, the moving element of which is mechanically coupled with the moving element of one of above mentioned devices 176, 177 and 178 causing relative moving between each other of at least one photosensitive element 174 of the detection device 173 placed on the moving element 10 and the spectrum 273 of the resultant light beam.

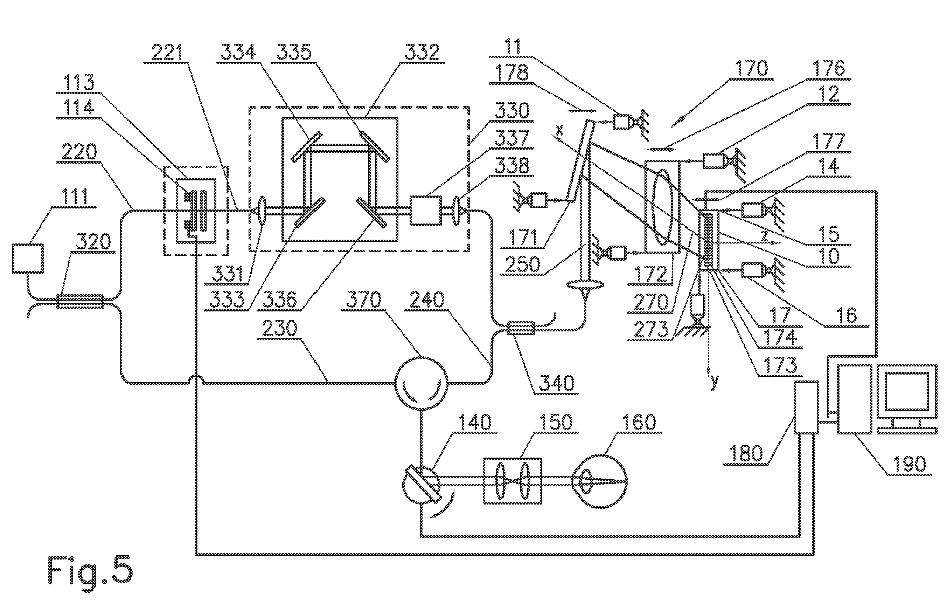

The presented invention, shown in Fig. 5, is an apparatus for imaging objects using optical Fourier domain tomography, also called an apparatus for imaging of objects in coherence light, which comprises a light source 111 and a fiber-coupler 320, which splits the light beam into at least one reference light beam 220 and at least one object light beam 230. The Fabry-Perot interferometer 113 that is fitted with a positioning device 114 and that can be aligned with the reference light beam or the object light beam forms or generates a reference optical frequency light comb 221 or an object optical frequency comb. In the way of the reference light beam is placed an optical arrangement 330 with an optical element 331 such as a collimator, a delay system 332, possibly a combination of four mirrors 333 - 336, a group of optical components 337 for compensation of dispersion in both arms of the apparatus and an optical element 338, such as a focusing lens for focussing the reference light beam 220. The object light beam 230 is directed onto the object 160 by a linear optical 3-way circulator 370 and the optical system 140 for changing beam direction, and by a set of optical elements 150 that forms the object light beam 230. The reference and object light beams 220, 230, 240 are directed to the fiber-coupler 340, and then to the spectrometer 170. The resultant light beam 250 is directed onto the dispersion device, for instance diffraction grating 171, from which as the spectrum 270 is imaged onto the detection device 173.

The apparatus for optical frequency domain tomography comprises the control system 180, a calculation unit 190 as well as an adjustment system of the apparatus for optical frequency domain tomography, which can be the device 176 to align the optical elements 172, the device 177 to control a position of the detection device 173 as well as the device 178 to control a position of the spectrometer relative to the resultant light beam 250, which comprises, among others, at least one working unit or an actuator, for instance the servomechanism 11, 12, 14, 16, which is mechanically coupled with the moving element of one of above mentioned devices 176, 177 and 178 causing relative moving between each other of at least one photosensitive element 174 of the detection device 173 placed on a supporting element 10 and the spectrum 273 of the resultant light beam.

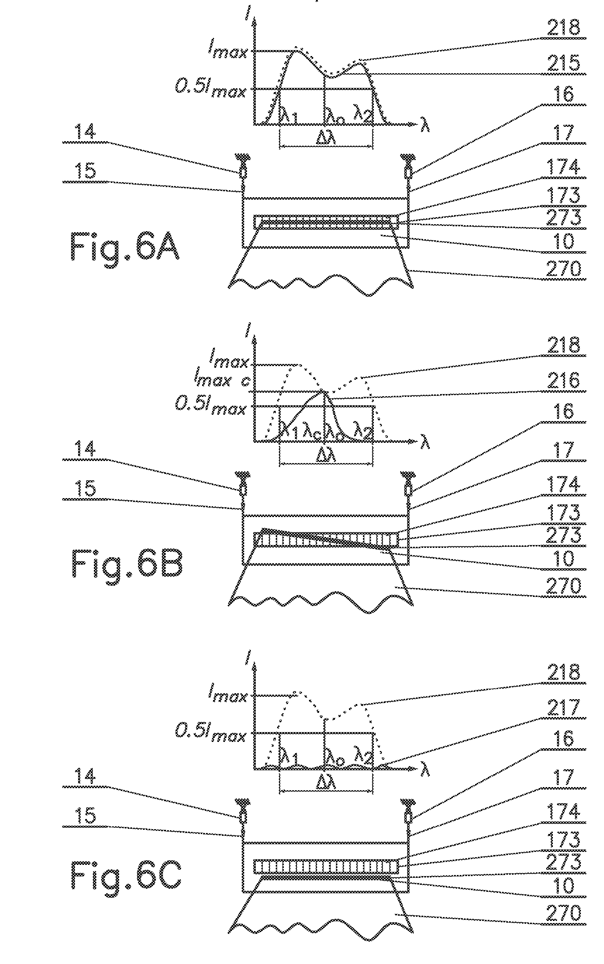

Independently of a version of the apparatus for imaging of objects by applying optical frequency domain tomography as well as a method for imaging of objects by applying optical frequency domain tomography, before examining the objects, the apparatus for imaging of objects should be adjusted. The biggest technical problem to be solved in the apparatus for imaging of objects by applying optical frequency domain tomography is achieving such position of the matrix of CCD camera, in which the image of the spectrum of the resultant light beam will be situated on the matrix most optimally. In one of the embodiments of the apparatus for imaging of objects by applying optical frequency domain tomography the image of the spectrum can be a line or a rectangle with its width much shorter than its length, as shown in Figs. 6A, 6B and 6C. Dependently on the degree of adjusting, the image of the spectrum can be in a correct position shown in Fig. 6A, in an oblique position shown in Fig. 6B or in a displacement position shown in Fig. 6C or in an oblique-displaced position, in which no part of the image is projected on the matrix of the detection device. To bring the image 273 of the spectrum to the correct position, a displacement or shifting or rotary devices 14, 16 can be used, whose elements can be moved to achieve a relative displacement of the spectrum image 273 and the detection device 173, called shortly a matrix. These displacement or shifting or rotary devices can be systems basing on a screw joint maintained by hand or displaced by different kind of mechanism controlled electrically, pneumatically or hydraulically. An adjusting system of the apparatus for imaging of objects by applying optical frequency domain tomography, which is the object of the present invention, comprises at least one servomechanism 11, 12, 13, 14, 16 jointed mechanically with a moving element of the dispersion device 171 being a prism or a diffracting grating, and/or the set of optical elements 172, and/or the detection device 173 causing a relative displacement of at least one photosensitive element of the detection device, also called a spectrum register or recorder of the spectrum, and the spectrum image, for instance in a plane perpendicularly situated to propagation direction of the of the resultant light beam.

Structure and a working principle of the adjusting system of the apparatus for imaging of objects will be described by way of example and with reference to the Figs. 6A, 6B and 6C, which show the adjusting system containing servomechanisms 14, 16, jointed or connected by a point of support or an articulated joint 15, 17 to the supporting element 10 of the detection device 173 created by photosensitive elements 174.

In one of the embodiments, the supporting element 10 of the detection device or photosensitive elements at one end, instead be jointed to one of the servomechanisms, is jointed to a stationary or adjustable element, whose position is handy adjusted. Yz-plane, in which the detection device containing photosensitive elements 174 is displaced, is situated perpendicularly to a propagation direction or an incidence direction of a split light beam 270. In case of an ideal and maximally adjusted optical spectrometer, shown in Fig. 6A, in which a signal has parameters, which suit the user expectation, that means, the signal has parameters allowing a correct work of the apparatus for imaging of objects, the image 273 of the spectrum, being a line, coincidences with the detection device 173 of the CCD camera, which has a shape of a line of photosensitive elements.

It should be noticed, that when the spectrum image has a shape other than a line, there is a possibility to find an axis of symmetry of the spectrum or a line almost similar to the axis of symmetry, which can be called a line of the spectrum image or matrix. In each case the structure and the working principle of the adjusting system of the apparatus for imaging of objects will be the same. In case shown in Fig. 6A, when the spectrum image 273 coincidences with the detection device 173, a curve 215 of light intensity / or energy versus wave length A registered by the detection device has a shape similar to a curve 218 of light intensity / or energy emitted by the light source versus wave length A or area under the curve of light intensity / or energy emitted by the light source achieves maximum possible area corresponding to the area under the curve of light intensity / or energy emitted by the light source after taking into account scattering loss. In this drawing there are shown *Iₘₐₓ* that corresponds to one thousand of units of account, *0.5Iₘₐₓ*, and *λ₁*, *λ₂*, at which light intensity / achieves a value of *0.5Iₘₐₓ* as well as λ*₀*, which is called a central wavelength of the light source waveband. As one thousand of units of account can be also taken a maximum of energy emitted by the light source, which is registered or recorded by the detection device or a maximum of a distribution curve of electric charge generated by the photosensitive elements of the detection device.

Fig. 6B shows a state, in which an electrical signal is present, but its parameters do not answer the expectation, that means, that the resultant light beam 270 has got a contact with the detection device 173, but the apparatus for imaging of objects by applying optical frequency domain tomography needs further adjusting. In this state a curve 216 of light intensity of registered spectrum has momentary maximum *I_{maxc}* at light wavelength *λ_{c}*, which is shifted in respect to the central wavelength λ₀ of the light source waveband, and which coincidences with a given photosensitive element, in other words, a pixel, for instance bearing a number 1308. When the momentary maximum *I_{maxc}* occurs at the central wavelength λ₀, change-over of direction rotation of the supporting element 10 of the photosensitive elements 174 can take place, and a point, at which the movement direction of the detection device is changed-over, is shortly called a change-over point or a switching over point. The change-over point can be different for different apparatus for imaging of objects by applying optical frequency domain tomography. In practice it is assumed that the change-over point, called also a centre of the light source waveband, at which, during clockwise movement of the detection device or its clockwise rotation of the detection device 173, called shortly a matrix, or in opposite direction, shortly counter-clockwise rotation, the light intensity / has approximately a constant value and the area under the curve of the light intensity changes in a small range. If the centre of the light source waveband appears near by the change-over point, for instance in the range of thirty pixels that answer thirty successive photosensitive elements, then each point appearing in this range of the light wavelength will be taken for a real change-over point.

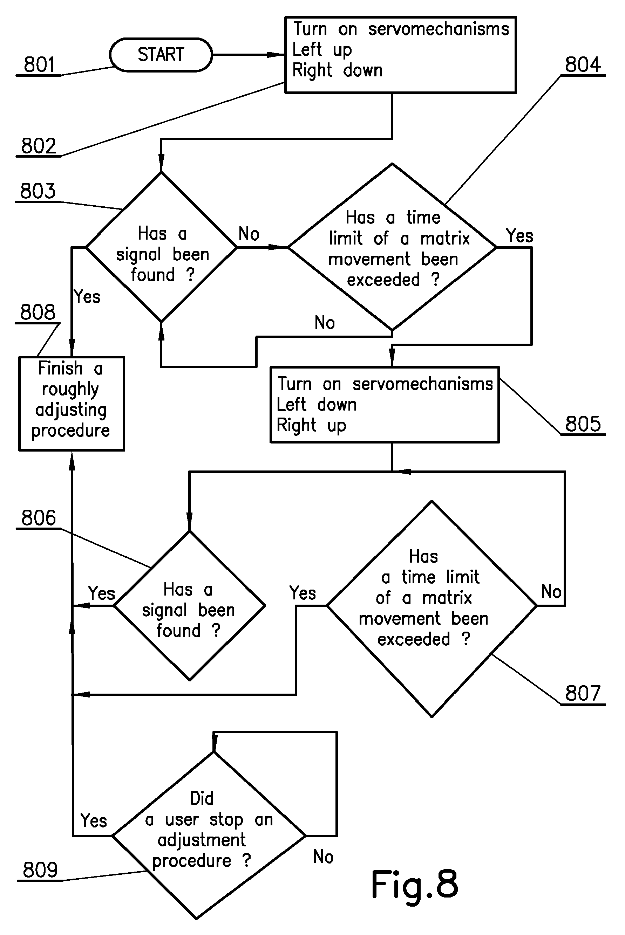

Additionally, the optical spectrometer can be completely out of adjustment, shown in Fig. 6C, in which no electrical signal can be found or the electrical signal generated by the detection device is so weak that can not be interpreted. The out of adjustment state also appears when not all necessary devices are switched on. When the optical spectrometer is not fully adjusted, the curve 217 of registered light spectrum shows a background, which answers a minimum spectrum or minimum registered power or energy or value of light radiation of surroundings registered by the detection device and which reaches the detection device from different light sources. To adjust the optical spectrometer, the adjusting system should undertake actions that correspond to the state, in which the spectrometer is. In the first step of adjusting it is found in which state the apparatus for imaging of objects is and corresponding algorithms of regulation or adjustment are undertaken. In one of the states, a preliminary roughly adjusting procedure shown in Fig. 8 starts on and the servomechanisms arrange the spectrum image in such position, in which the spectrum image has a contact with the detection device. In the case, when during the preliminary roughly adjusting procedure the electrical signal reaches parameters that answer presumptions, the procedure of adjusting the spectrometer is finished. When a possible maximal adjustment of the apparatus for imaging of objects is required, firstly a search is undertaken to determine parameters that can be reached for a given apparatus for imaging of objects. This is realized when to high parameters are required or set. As the preliminary roughly adjusting procedure is convergent, the apparatus for imaging of objects reaches the maximum parameters and will oscillate around them. Then it is determined if the maximum parameters are reached with previously assumed accuracy, that means, whether parameters of registration of the spectrum image achieve the parameters determined by the manufacturer of the apparatus for optical frequency domain tomography or recognized by a user as sufficient for registration of the spectrum. For example, when the curve of light intensity / versus a wave length A of the registered spectrum image reaches approximately a shape of the curve of light intensity / versus a wave length A of the light source, for instance in 99%, or area under the curve of energy or light intensity / registered by the detection device versus wave length A achieves with accuracy, for instance 1%, area corresponding to the area under the curve of energy or light intensity / emitted by the light source versus wave length A after taking into account scattering loss.

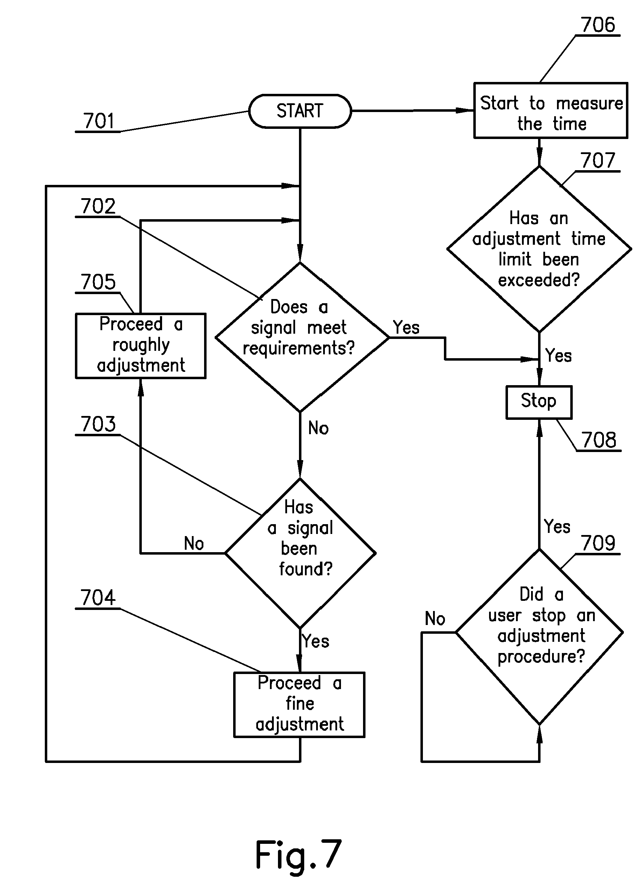

If current values of energy or light intensity or parameters answer maximum expected values with assumed accuracy, the adjusting system finishes the adjustment, checks whether the reached parameters of adjustment answer the expectations and informs the users of results and their interpretation. When the work of the apparatus for imaging of objects will be disturbed, the adjustment procedure or algorithm will proceed to reach the correct conditions of the work. An adjustment method has been shown by means of flow charts in Figs. 7, 8 and 9.

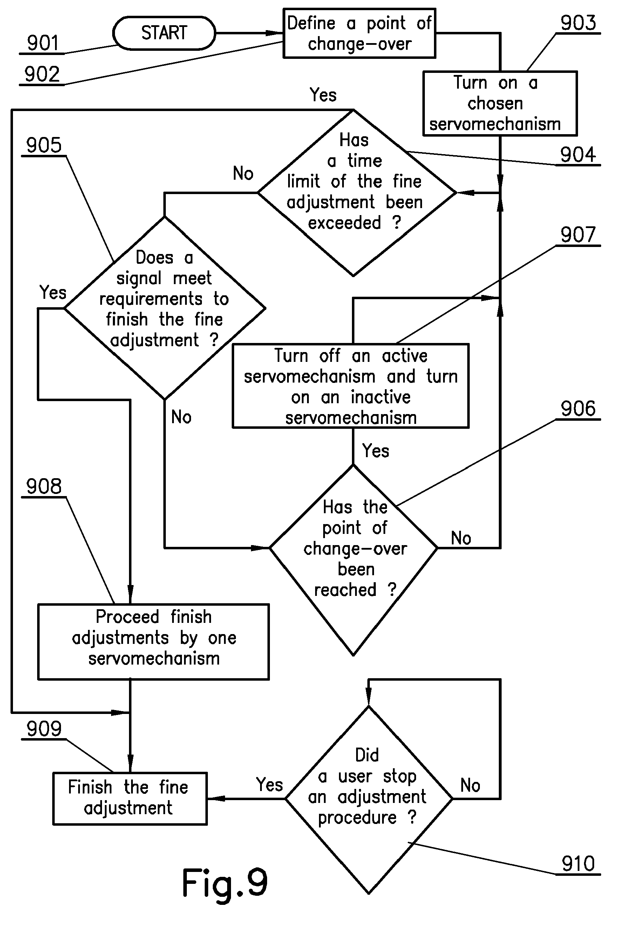

Fig. 7 shows a flow diagram of an adjusting procedure of the apparatus for imaging of objects, which starts in step 701. Simultaneously, in step 706, the procedure starts to measure the time of adjustment and if the adjustment time limit has passed, the adjustment is finished in step 708. Otherwise, in step 702, it is checked if an electric signal generated by the detection device meets requirements. In case, when the electric signal generated by the detection device meets the requirements, the adjustment is finished in step 708. If not, then, in step 703 it is checked if the detection device generates the electric signal and if the signal has been found at all. When there is no signal, a preliminary roughly adjustment shown in Fig. 8 is conducted in step 705, otherwise, when the electric signal has been found, a fine adjustment takes place in step 704, which is shown as a flow diagram in Fig. 9. The user can stop the procedure at any time, and if the user decides to finish the procedure, the procedure is broken by the user in step 709.

The preliminary roughly adjustment, shown in Fig. 8, starts in step 801, when no electric signal has been found in step 703 of the procedure shown in Fig. 7. Next, in step 802, one of servomechanisms is turn on, for instance the left servomechanism, which moves up the supporting element of the detection device, or the right servomechanism, which moves down the supporting element of the detection device or the matrix. During movement of the detection device by one of the servomechanisms, in step 803, it is checked if the detection device generates the electric signal, when the detection device stars to generate the electric signal, the preliminary roughly adjustment is finished and the main adjustment procedure is continued in step 702 of Fig. 7. If the time limit of the roughly adjustment has passed, that is checked in step 804, a change-over of the movement of the detection device takes place in step 805. For instance, when the left servomechanism has moved up the supporting element of the detection device, or the right servomechanism has moved down the supporting element of the detection device, then, the left servomechanism moves down the supporting element of the detection device, or the right servomechanism moves up the supporting element of the detection device in step 805. In step 806 it is checked if the detection device generates the electric signal. When the detection device generates the electric signal, the preliminary roughly adjustment is finished in step 808 and the procedure moves to the step 702 of the main adjustment procedure shown in Fig. 7. When the time limit of the matrix movement has passed, that is checked in step 807, the preliminary roughly adjustment is finished in step 808 and the procedure moves to the step 702, where the adjustment time of this procedure means a movement time of a moved end of the supporting element from one lateral position to the other lateral position, as well as a movement time of the detection device from one lateral position to the other lateral position to assure that the detection device reaches both lateral positions. The user can stop the procedure at any time, and if the user decides to finish the procedure, the procedure is broken by the user in step 809.

Fig. 9 shows a flow diagram of a fine adjustment procedure, which starts in step 901, when the electric signal has been found in step 704 of Fig. 7. In step 902 a point of change-over of the movement of the detection device is defined, and then in step 903, a chosen servomechanism is turn on, the right or left servomechanism, which moves up or down the supporting element. If the electric signal generated by the detection device meets the requirements to finish the fine adjustment, what is checked in 905, and then a final adjustment procedure using one servomechanism starts in step 908. The final adjustment procedure is finished in step 909. In case when the electric signal generated by the detection device does not meet the requirements to finish the fine adjustment, in step 906 it is checked whether the point of change-over is reached, and after its crossing, an active servomechanism is turn off and an inactive servomechanism is turn on in step 907. Next, in step 905, it is checked again whether the electric signal generated by the detection device meets the requirements to finish the fine adjustment. The user can stop the procedure at any time, and if the user decides to finish the procedure, the procedure is broken by the user in step 910. The procedure is also finished when the adjustment time limit has passed. The adjustment time is measured in step 904.

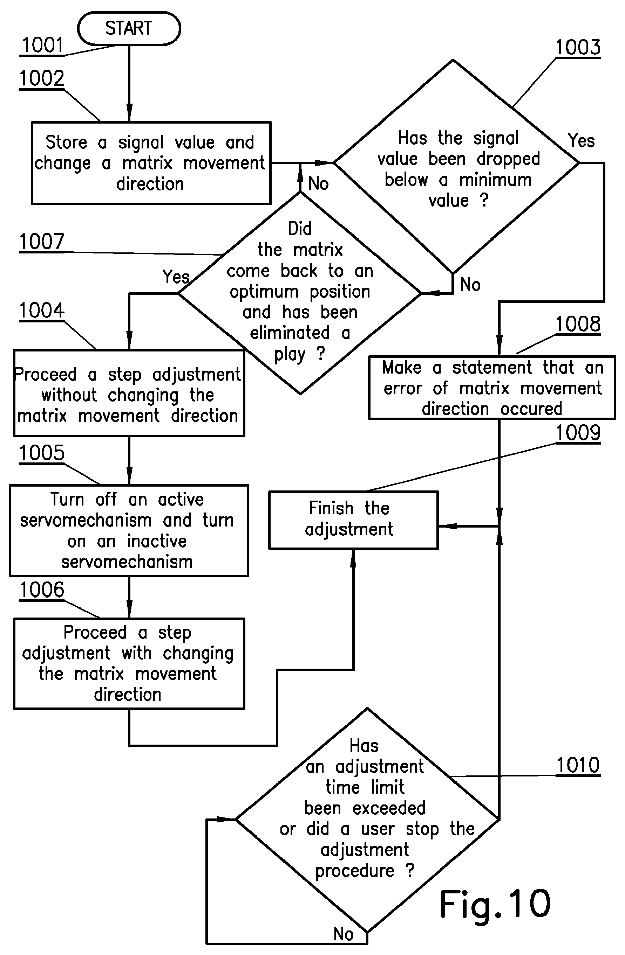

Fig. 10 shows a flow diagram of the final adjustment using one of servomechanisms, also called a final adjustment with one parameter, which starts in step 1001, when the fine adjustment has been finished. In step 1002, a signal value is stored and a matrix movement direction is changed-over. In step 1003 it is checked if the signal drops below a minimum limit value, for instance below three hundred of units of account, and in step 1007, if the matrix comes back to an optimum position and a play or running clearance has been eliminated. When the matrix nearly reaches the optimum position, the input value or the average value of the electric signal generated by the detection device is stored in step 1004 and a step adjustment procedure is started. In step 1005 the active servomechanism is turn off and the inactive servomechanism is turn on to proceed the step adjustment with changing the matrix movement direction in step 1006, after which the adjustment procedure is finished in step 1009. When the signal drops below the minimum limit value, in step 1008 it is stated that an error occurred in respect to the matrix movement direction. The adjustment is finished in step 1009. The user can stop the procedure at any time, and if the user decides to finish the procedure, the procedure is broken by the user in step 1010. The procedure is also finished when the adjustment time limit has passed. The adjustment time is measured in step 1010.

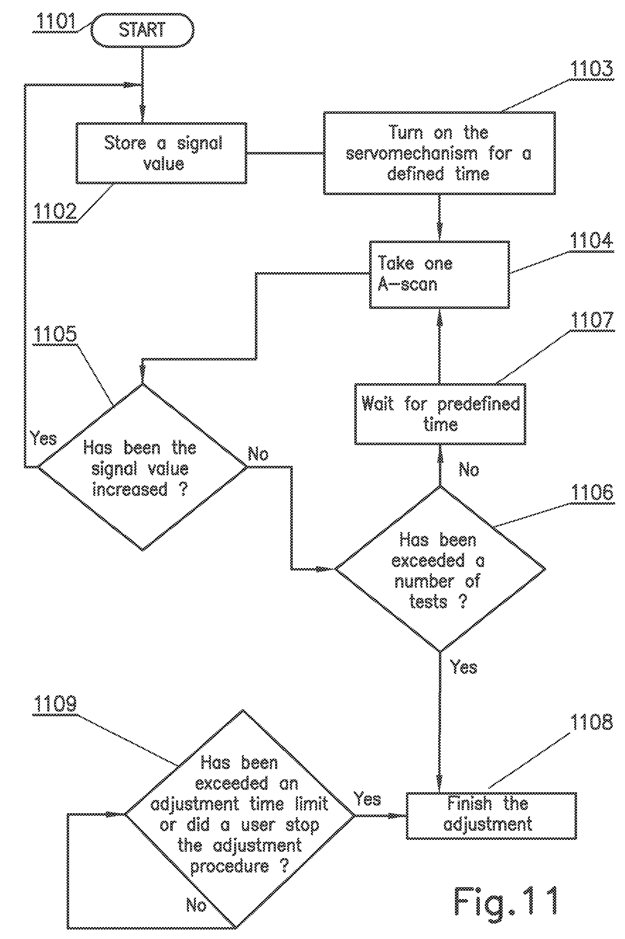

Fig. 11 shows a flow diagram of the step adjustment, which starts in step 1101. In step 1102, a signal value is stored and the servomechanism is turn on for short time, for example for 300 ms, in step 1103. Next, the servomechanism is turn off and the value of the electric signal is measured after taking one A-scan in step 1104. In step 1105 it is checked whether the value of the electric signal has increased, and if yes, the adjustment is continued in step 1102. If the value of the electric signal did not increase, then in case when the number of tests set previously has been exceeded that is checked in step 1106, for example after taking twenty A-scans, the step adjustment is finished in step 1108. When the number of tests set previously has been not exceeded, then when passes a period of time previously set, for example 30 ms, measured in step 1107, the procedure is continued in step 1104 and a next A-scan is taken. The user can stop the procedure at any time, and if the user decides to finish the procedure, the procedure is broken by the user. The procedure is also finished when the adjustment time limit has passed. The adjustment time is measured in step 1109.

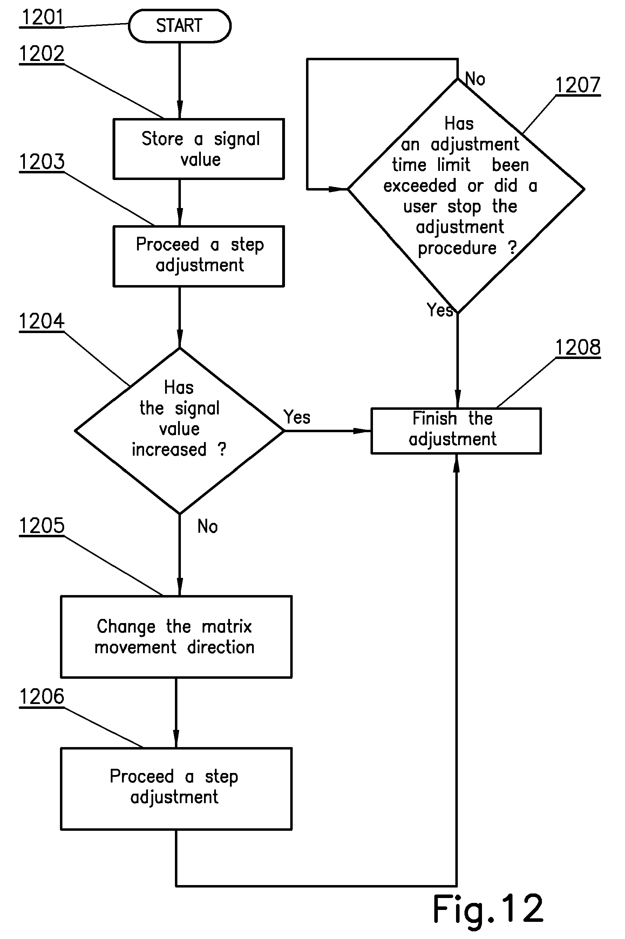

Fig. 12 shows a flow diagram of a step adjustment with changing-over direction of matrix rotation or movement, which starts in step 1201. In step 1202, a signal value is stored and then, in step 1203, the step adjustment, shown in Fig. 11, is proceeded. In step 1204 it is checked whether the value of the electric signal has increased and if no, then in step 1205, direction of rotation of the matrix is changed-over and the step adjustment, shown in Fig. 11, is proceeded. After the step adjustment, the step adjustment with changing-over direction of rotation is finished. The user can stop the step adjustment procedure at any time, and if the user decides to finish the step adjustment procedure, the step adjustment procedure is broken by the user in step 1208. The step adjustment procedure is also finished when the adjustment time limit has passed. The adjustment time is measured in step 1207.

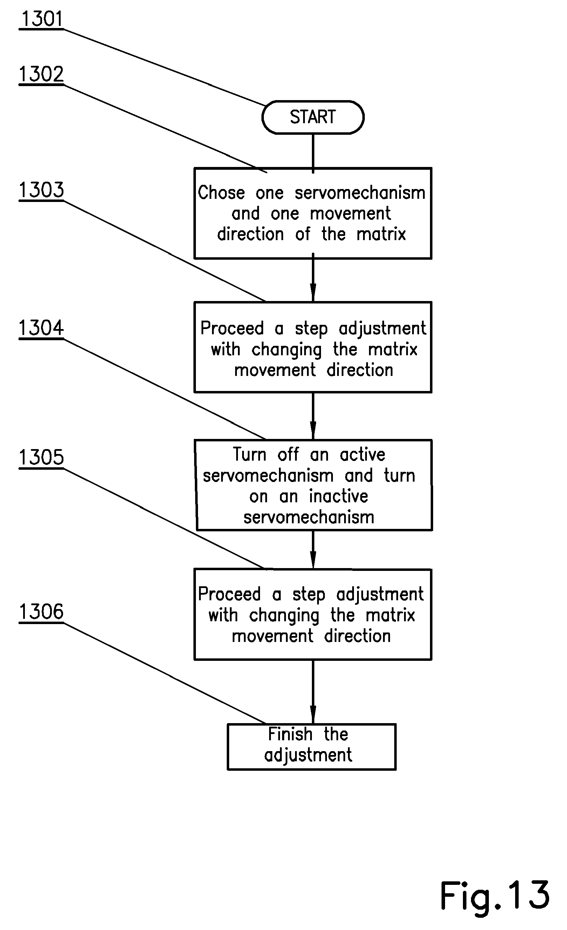

Fig. 13 shows a flow diagram of the step adjustment with changing of the servomechanism, which starts in step 1301. In step 1302, one of servomechanisms and one direction of the matrix movement are chosen, after which the step adjustment with changing-over matrix direction of rotation is proceeded in step 1303. In step 1304 is turn off an active servomechanism and turn on an inactive servomechanism, and next, in step 1305, the step adjustment with changing-over matrix direction of rotation is proceeded. The step adjustment with changing of the servomechanism is finished in step 1306.

## Claims

1. An apparatus for imaging of objects by applying optical frequency domain tomography and provided with an adjusting system for setting a relative position of photosensitive elements and a spectrum image, and comprising at least one device for spectral analysis of a light beam with a dispersion device, a set of optical elements, a detection device of the spectrum **characterised in that** the adjusting system is an automatically controlled device causing a relative displacement of at least one photosensitive element (174) of the detection device (173) of the spectrum and the spectrum image between each other, and comprising at least one actuator acting on the dispersion device (171) and/or the set of optical elements (172) and/or the detection device (173) or a displaceable element of the dispersion device (171) and/or the set of optical elements (172) and/or the detection device (173) and moving of which causes the relative displacement of at least one photosensitive element (174) of the detection device (173) of the spectrum and the spectrum image of the resultant light beam (270) between each other.

2. The apparatus for imaging of objects by applying optical frequency domain tomography according to claim 1 **characterised in that** the relative displacement of at least one photosensitive element (174) of the detection device (173) of the spectrum and the spectrum image between each other takes place in a plane situated perpendicularly to propagation direction of the resultant light beam (270).

3. The apparatus for imaging of objects by applying optical frequency domain tomography according to claim 1 or 2 **characterised in that** the actuator acting on the dispersion device (171) and/or the set of optical elements (172) and/or the detection device (173) or a displaceable element of the dispersion device (171) and/or the set of optical elements (172) and/or the detection device (173) is a mechanism comprising at least one servomechanism (11, 12, 13, 14, 16) controlled by electric signal, and a movable element of the servomechanism is connected mechanically to the dispersion device (171) and/or the set of optical elements (172) and/or the detection device (173) or a displaceable element of the dispersion device (171) and/or the set of optical elements (172) and/or the detection device (173).

4. The apparatus for imaging of objects by applying optical frequency domain tomography according to claim 1 or 2 **characterised in that** the actuator acting on the dispersion device (171) and/or the set of optical elements (172) and/or the detection device (173) or a displaceable element of the dispersion device (171) and/or the set of optical elements (172) and/or the detection device (173) is a mechanism comprising at least one element (11, 12, 13, 14, 16) connected to the dispersion device (171) and/or the set of optical elements (172) and/or the detection device (173) or a displaceable element of the dispersion device (171) and/or the set of optical elements (172) and/or the detection device (173) and the element is displaceabled by magnetic or electromagnetic field or a force evoked by pressure of fluid, gas or gas mixture, and is connected to the dispersion device (171) and/or the set of optical elements (172) and/or the detection device (173) or to a displaceable element of the dispersion device (171) and/or the set of optical elements (172) and/or the detection device (173).

5. A system for adjusting an apparatus for imaging of objects by applying optical frequency domain tomography provided with at least one device (170) for spectral analysis of a light beam with a dispersion device (171), a set of optical elements (172) and a detection device (173) of the spectrum **characterised in that** it is an automatically controlled device causing a relative displacement of at least one photosensitive element (174) of the detection device (173) of the spectrum and the spectrum image between each other and comprising at least one actuator acting on the dispersion device (171) and/or the set of optical elements (172) and/or the detection device (173) or a displaceable element of the dispersion device (171) and/or the set of optical elements (172) and/or the detection device (173) and movement of which causes the relative displacement of at least one photosensitive element (174) of the detection device (173) of the spectrum and the spectrum image between each other.

6. The system for adjusting an apparatus for imaging of objects by applying optical frequency domain tomography according to claim 5 **characterised in that** the relative displacement of at least one photosensitive element (174) of the detection device (173) of the spectrum and the spectrum image between each other takes place in a plane situated perpendicularly to propagation direction of the resultant light beam (270).

7. The system for adjusting an apparatus for imaging of objects by applying optical frequency domain tomography according to claim 5 or 6 **characterised in that** the actuator acting on the dispersion device (171) and/or the set of optical elements (172) and/or the detection device (173) or a displaceable element of the dispersion device (171) and/or the set of optical elements (172) and/or the detection device (173) is a mechanism comprising at least one servomechanism (11, 12, 13, 14, 16) controlled by electric signal, and a movable element of the servomechanism is connected mechanically to the dispersion device (171) and/or the set of optical elements (172) and/or the detection device (173) or to a displaceable element of the dispersion device (171) and/or the set of optical elements (172) and/or the detection device (173).

8. A method for adjusting apparatus for optical frequency domain tomography provided with an adjusting system, at least one device for spectral analysis of a light beam formed from a reference light beam and an object light beam, a dispersion device for splitting a resultant light beam into a spectrum of the resultant light beam, a set of optical elements for forming and/or directing the resultant light beam and a detection device of the spectrum with at least one photosensitive element for registering the spectrum of resultant light beam **characterised in that** at least one photosensitive element of the detection device of the resultant light beam and an image of the spectrum is displaced relatively each other using at least one actuator acting on the dispersion device and/or the set of optical elements and/or the detection device or a displaceable element of the dispersion device and/or the set of optical elements and/or the detection device and movement of which causes the relative displacement of at least one photosensitive element of the detection device of the spectrum and the spectrum image of the resultant light beam between each other.

9. The method for adjusting apparatus for optical frequency domain tomography according to claim 8 **characterised in that** when displacing at least one photosensitive element of the detection device of the resultant light beam and the image of the spectrum relatively each other using the actuator, the detection device and a line of the image of the spectrum is brought to cross each other, and that a point of change-over is determined at the detection device and direction of rotation of at least one photosensitive element relatively to the image of the resultant light beam or rotation of the image of the resultant light beam relatively to at least one photosensitive element is changed after crossing the point of change-over by the line of the image of the spectrum, and that changing the direction of rotation is being done until parameters of registration of the spectrum determined by a manufacturer of the apparatus for optical frequency domain tomography or recognized by a user as sufficient for registration of the spectrum are achieved.

10. The method for adjusting apparatus for optical frequency domain tomography according to claim 8 **characterised in that** the displacing of at least one photosensitive element relatively to the image of the resultant light beam is being done until an electric signal is generated by the detection device, and after determining a point of change-over on the detection device, direction of rotation of at least one photosensitive element of the detection device relatively to the image of the resultant light beam or rotation of the image of the resultant light beam relatively to at least one photosensitive element of the detection device is changed at every crossing the point of change-over by the line of the image of the spectrum, and changing of the direction of rotation is being done until a curve of light intensity / or energy registered by the detection device versus wave length λ achieves a shape corresponding to a shape of a curve of light intensity / or energy of the light emitted by the light source versus wave length λ or area under the curve of light intensity / or energy registered by the detection device versus wave length λ achieves area corresponding to the area under the curve of light intensity / emitted by the light source after taking into account scattering loss.
